# EUROPEAN PATENT SPECIFICATION

(11) **EP 3 942 999 B1**
(45) Date of publication and mention of the grant of the patent: **11.09.2024**
(21) Application number: 21184249.7
(22) Date of filing: 07.07.2021
(51) Int. Cl.: A61B 5/021, A61B 5/022, A61B 5/024, A61B 5/00, A61B 5/02, A61B 5/1495

(54) **APPARATUS AND METHOD FOR ESTIMATING BIO-INFORMATION**
VORRICHTUNG UND VERFAHREN ZUR SCHÄTZUNG VON BIOINFORMATIONEN
APPAREIL ET PROCÉDÉ D'ESTIMATION D'INFORMATIONS BIOLOGIQUES

(30) Priority: 23.07.2020 KR 20200091509
(43) Date of publication of application: 26.01.2022
(73) Proprietor: Samsung Electronics Co., Ltd., Suwon-si, Gyeonggi-do 16677 (KR)
(72) Inventor: CHOI, Jin Woo, Gyeonggi-do (KR); PARK, Sang Yun, Gyeonggi-do (KR); LIM, Hye Rim, Gyeonggi-do (KR); KANG, Jae Min, Seoul (KR); NOH, Seung Woo, Gyeonggi-do (KR)
(74) Representative: Grünecker Patent- und Rechtsanwälte PartG mbB

(56) References cited:
- US-A1- 2015 374 249
- US-A1- 2020 085 320
- US-A1- 2020 093 377

## Description

### BACKGROUND

### 1. Field

The disclosure relates to an apparatus and method for estimating bio-information, and technology for cuffless blood pressure estimation.

### 2. Description of Related Art

General techniques for extracting cardiovascular characteristics, such as blood pressure, and the like, without using a pressure cuff include a pulse transit time (PTT) method and a pulse wave analysis (PWA) method.

The pulse transit time (PTT) method is a method of extracting cardiovascular characteristics by analyzing the shape of a photoplethysmography (PPG) signal or a body surface pressure signal from a peripheral part of the body, such as a fingertip, a radial artery, or the like. The blood ejected from the left ventricle causes reflection at areas of large branches, such as the renal arteries and the iliac arteries, and the reflection affects the shape of the pulse wave or body pressure wave measured at the peripheral part of the body. Thus, by analyzing this shape, arterial stiffness, arterial age, aortic artery pressure waveform, or the like, can be inferred.

The pulse wave velocity (PWV) method is a method of extracting cardiovascular characteristics, such as arterial stiffness, blood pressure, or the like, by measuring a pulse wave transmission time. In this method, a delay (a pulse transit time (PTT)) between an R-peak (left ventricular contraction interval) of an electrocardiogram (ECG) and a peak of a PPG signal of a finger or the radial artery is measured by measuring the ECG and PPG signals of the peripheral part of the body, and by calculating a velocity at which the blood from the heart reaches the peripheral part of the body by dividing an approximate length of the arm by the PTT
US 2020/093377 A1 discloses a method and an apparatus for estimating bio-information. Said apparatus for estimating bio-information includes a sensor part comprising a pulse wave sensor array, a load sensor and a processor. The pulse wave sensor array is hereby configured to detect a pulse wave signal when an object contacts a contact surface of the sensor part, and the load sensor is configured to detect a first contact load applied by the object to the contact surface. Further, the processor is configured to obtain a contact load distribution of the contact surface based on the pulse wave signal, obtain a second contact load at each position of the contact surface based on the contact load distribution and the first contact load and estimate bio-information based on the second contact load and the pulse wave signal. The estimating the bio-information includes setting a region of interest based on at least one of a blood vessel distribution of the object, an arrangement of a pulse wave sensor array, a specific position of the contact surface of the sensor and the second contact load at each position of the contact surface. Moreover, in one example of US 2020/093377 A1, it is disclosed that once a pulse wave signal is detected at each position, the quality of each pulse wave signal is evaluated and a region of interest is set based on the evaluation. Hereby, the quality of each pulse wave signal is e.g. evaluated based on a maximum amplitude value of each pulse wave signal, a difference between a maximum amplitude value and a minimum amplitude value, an average amplitude value etc.
US 2020/085320 A1 relates to a bio-signal acquiring apparatus that includes a sensor part and a signal processor. The sensor part hereby comprises a bio-signal sensor, a load sensor, and an ultrasonic sensor array. The bio-signal sensor is configured to detect a bio-signal of an object that comes into contact with the sensor part, the load sensor is configured to detect a contact load of the object and the ultrasonic sensor array is configured to detect a contact load distribution of the object. The signal processor is configured to obtain a contact load of the object at a region of interest based on the contact load and the contact load distribution, and configured to output the contact load of the object at the region of interest and the bio-signal. In this context, US 2020/085320 A1 also mentions setting a region of interest by considering a distribution of blood vessels of an object.
US 2015/0374249 A1 provides a personal hand-held monitor (PHHM) which comprises a signal acquisition device for acquiring signals which can be used to derive a measurement of a subject's blood pressure (BP), the signal acquisition device being integrated with a personal hand-held computing device (PHHCD). The signal acquisition device comprises a blood flow occlusion means adapted to be pressed against one side only of a body part or to have one side only of a body part pressed against it, a means for measuring the pressure applied by or to the body part, and a means for detecting the flow of blood through the body part in contact with the blood flow occlusion means. The blood flow occlusion means comprises at least part of an external surface of the PHHM and wherein the pressure is sensed by means of a flexible and essentially incompressible gel in which is immersed a pressure sensor. The pressure sensor is adapted to provide electrical signals to the processor of the PHHCD.

The invention is defined by the subject matter of the independent claims. Preferred embodiments are defined by the dependent claims.

### SUMMARY

According to an aspect of an example embodiment, an apparatus for estimating bio-information of a user includes a pulse wave sensor configured to measure a plurality of pulse wave signals from an object of the user; a position sensor configured to obtain sensor position information identifying a sensor position on the object for each of the plurality of pulse wave signals, based on the pulse wave sensor measuring each of the plurality of pulse wave signals; and a processor configured to estimate first bio-information at each sensor position based on each of the plurality of pulse wave signals; and estimate second bio-information based on a blood vessel position of the object, each sensor position, and the first bio-information at each sensor position.

Based on the object being in contact with the pulse wave sensor, the position sensor is further configured to obtain the sensor position information based on an image of the object which is captured by an external capturing device.

The position sensor may include a fingerprint sensor configured to obtain a fingerprint image, and the position sensor is further configured to obtain the sensor position information based on the fingerprint image obtained by the fingerprint sensor based on the object being in contact with the pulse wave sensor.

Based on the object being in contact with the pulse wave sensor, the position sensor is further configured to obtain the sensor position information based on pre-defined measurement position information of the pulse wave sensor.

The apparatus may include a blood vessel position sensor configured to obtain the blood vessel position information of the object based on at least one of an optical image, an ultrasonic image, a magnetic resonance imaging (MRI) image, and a photoacoustic image, of the object which are obtained by an external device.

The apparatus may include a blood vessel position sensor which includes an ultrasonic sensor configured to transmit an ultrasonic wave to the object and receive a signal reflected from the object, and obtain the blood vessel position information of the object based on an ultrasonic image obtained by the ultrasonic sensor.

The processor is further configured to generate a calibration graph by plotting estimated bio-information values at each sensor position against a relative distance of each sensor position from the blood vessel position of the object; and based on performing curve fitting, obtain a final estimated bio-information value based on the calibration graph.

The processor is further configured to obtain a bio-information value at a point, corresponding to the blood vessel position of the object in the calibration graph, as the second bio-information.

The apparatus may include a force sensor configured to measure a force applied by the object to the pulse wave sensor; or a pressure sensor configured to measure a pressure applied by the object to the pulse wave sensor.

The processor is further configured to generate an oscillogram based on each of the plurality of pulse wave signals and the force measured by the force sensor or the pressure measured by the pressure sensor; and estimate the first bio-information at each sensor position by using the oscillogram.

The bio-information comprises one or more of blood pressure, vascular age, arterial stiffness, aortic pressure waveform, vascular compliance, stress index, fatigue level, skin age, and skin elasticity.

A method of estimating bio-information of a user may include measuring a plurality of pulse wave signals from an object; obtaining sensor position information identifying a sensor position on the object for each of the plurality of pulse wave signals, based on a pulse wave sensor measuring each of the plurality of pulse wave signals; estimating first bio-information at each sensor position based on each of the plurality of pulse wave signals; and estimating second bio-information based on a blood vessel position of the object, each sensor position, and the first bio-information at each sensor position.

The estimating of the second bio-information comprises generating a calibration graph by plotting first estimated bio-information values at each sensor position against a relative distance of each sensor position from the blood vessel position of the object, and by performing curve fitting, obtaining a second estimated bio-information value based on the calibration graph.

The estimating of the second bio-information comprises obtaining a bio-information value at a point, corresponding to the blood vessel position of the object in the calibration graph, as the second estimated bio-information value.

The method may include measuring a force or a pressure applied by the object to the pulse wave sensor.

The estimating of the first bio-information at each sensor position may include generating an oscillogram based on each of the plurality of pulse wave signals and the force or the pressure, and estimating the first bio-information at each sensor position by using the oscillogram.

According to an aspect of an example embodiment, an apparatus for estimating bio-information of a user may include a pulse wave sensor configured to measure a plurality of pulse wave signals from an object of the user; a position sensor configured to obtain sensor position information identifying a sensor position on the object for each of the plurality of pulse wave signals, based on the pulse wave sensor measuring each of the plurality of pulse wave signals; and a processor configured to estimate first bio-information at each sensor position based on each of the plurality of pulse wave signals; determine one of a plurality of virtual blood vessel positions as a blood vessel position of the object based on the first bio-information at each sensor position; and estimate second bio-information based on the blood vessel position of the object, each sensor position, and the first bio-information at each sensor position.

Based on a difference between first bio-information values at each sensor position, the processor is further configured to determine the one of the plurality of virtual blood vessel positions as the blood vessel position of the object.

A virtual blood vessel position is set for each of a plurality of groups which are pre-classified based on the difference between the first bio-information values at each sensor position obtained from a plurality of users.

The processor is further configured to determine a group, to which the difference belongs, among the plurality of groups; and determine a virtual blood vessel position, pre-defined for the determined group, as the blood vessel position of the object.

The processor is further configured to generate a calibration graph by plotting the first bio-information values at each sensor position against a relative distance of each sensor position from the blood vessel position of the object; and obtain a second bio-information value based on the calibration graph.

The processor is further configured to obtain a bio-information value at a point, corresponding to the blood vessel position of the object in the calibration graph, as the second bio-information value.

The apparatus may include a force sensor configured to measure a force applied by the object to the pulse wave sensor; or a pressure sensor configured to measure a pressure applied by the object to the pulse wave sensor.

The processor may generate an oscillogram based on each of the plurality of pulse wave signals and the force or the pressure measured by the force sensor or the pressure sensor; and estimate the first bio-information at each sensor position by using the oscillogram.

A method of estimating bio-information of a user may include measuring a plurality of pulse wave signals from an object of the user; obtaining sensor position information identifying a sensor position on the object for each of the plurality of pulse wave signals, based on a pulse wave sensor measuring each of the plurality of pulse wave signals; estimating first bio-information at each sensor position based on each of the plurality of pulse wave signals; determining one of a plurality of virtual blood vessel positions as a blood vessel position of the object, based on the first bio-information at each sensor position; and estimating second bio-information based on the blood vessel position of the object, each sensor position, and the first bio-information at each sensor position.

The determining of the one of a plurality of virtual blood vessel positions as the blood vessel position of the object comprises, based on a difference between first bio-information values at each sensor position, determining the one of the plurality of virtual blood vessel positions as the blood vessel position of the object.

The determining of the one of a plurality of virtual blood vessel positions as the blood vessel position of the object comprises determining a group, to which the difference belongs, among a plurality of groups; and determining a virtual blood vessel position, pre-defined for the determined group, as the blood vessel position of the object.

The estimating of the second bio-information comprises generating a calibration graph by plotting the first bio-information values at each sensor position against a relative distance of each sensor position from the determined blood vessel position of the object; and obtaining a second bio-information value based on the calibration graph.

The method may include measuring a force or a pressure applied by the object to the pulse wave sensor.

The estimating of the first bio-information at each sensor position comprises generating an oscillogram based on each of the pulse wave signals and the force or the pressure; and estimating the first bio-information at each sensor position by using the oscillogram.

### BRIEF DESCRIPTION OF THE DRAWINGS

The above and other aspects, features, and advantages of certain embodiments of the present disclosure will be more apparent from the following description taken in conjunction with the accompanying drawings, in which:
FIGS. 1 to 3 are block diagrams illustrating an apparatus for estimating bio-information according to embodiments of the present disclosure;
FIG. 4 is an example of a configuration of a processor of the apparatus for estimating bio-information illustrated in FIGS. 1 to 3;
FIGS. 5A and 5B are diagrams explaining an example of estimating blood pressure using oscillometry;
FIGS. 6A and 6B are diagrams explaining an example of calibrating blood pressure based on blood vessel positions of an object;
FIG. 7 is a block diagram illustrating an apparatus for estimating bio-information according to another embodiment of the present disclosure;
FIG. 8 is a diagram illustrating an example of a configuration of a processor of FIG. 7;
FIGS. 9A to 9C are diagrams explaining an example of calibrating blood pressure based on a virtual blood vessel position;
FIG. 10 is a block diagram illustrating an apparatus for estimating bio-information according to yet another embodiment of the present disclosure;
FIG. 11 is a flowchart illustrating a method of estimating bio-information according to an embodiment of the present disclosure;
FIG. 12 is a flowchart illustrating a method of estimating bio-information according to another embodiment of the present disclosure;
FIG. 13 is a diagram illustrating an example of a wearable device; and
FIG. 14 is a diagram illustrating an example of a smart device.

### DETAILED DESCRIPTION

Details of example embodiments are included in the following detailed description and drawings. Advantages and features of the present disclosure, and a method of achieving the same will be more clearly understood from the following embodiments described in detail with reference to the accompanying drawings. Throughout the drawings and the detailed description, unless otherwise described, the same drawing reference numerals will be understood to refer to the same elements, features, and structures.

It will be understood that, although the terms "first," "second," etc. may be used herein to describe various elements, these elements should not be limited by these terms. These terms are used to distinguish one element from another. Also, the singular forms of terms are intended to include the plural forms of the terms as well, unless the context clearly indicates otherwise. It will be further understood that when an element is referred to as "comprising" another element, the element is intended not to exclude one or more other elements, but to further include one or more other elements, unless explicitly described to the contrary. In the following description, terms such as "unit" and "module" indicate a unit for processing at least one function or operation and the unit may be implemented by using hardware, software, or a combination thereof.

Hereinafter, embodiments of an apparatus and method for estimating bio-information will be described in detail with reference to the accompanying drawings.

Various embodiments of the apparatus for estimating bio-information may be mounted in terminals such as a smart phone, a tablet personal computer (PC), a desktop computer, a laptop computer, etc., wearable devices, and the like. In this case, examples of the wearables devices may include a smartwatch type wearable device, a bracelet type wearable device, a wristband type wearable device, a ring type wearable device, a glasses type wearable device, or a headband type wearable device, etc., but the wearable devices are not limited thereto.

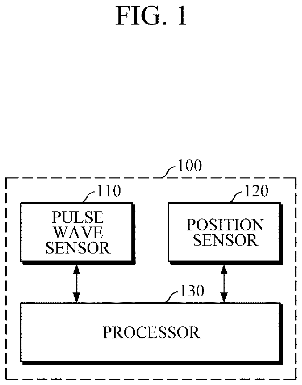

FIGS. 1 to 3 are block diagrams illustrating an apparatus for estimating bio-information according to embodiments of the present disclosure.

Referring to FIG. 1, the apparatus 100 for estimating bio-information includes a pulse wave sensor 110, a position sensor 120, and a processor 130.

The pulse wave sensor 110 measures a photoplethysmography (PPG) signal (hereinafter referred to as a "pulse wave signal") from an object. In this case, the object may be a body area which may be in contact with the pulse wave sensor 110, and may be a body part at which pulse waves may be easily measured based on PPG signals. For example, the object may be a finger where blood vessels are densely located, but the object is not limited thereto and may be an area on the wrist that is adjacent to the radial artery, or a distal portion of the body, such as an upper portion of the wrist, toes, etc., where veins or capillaries are located.

The pulse wave sensor 110 may include one or more light sources for emitting light onto the object, and one or more light receivers which are disposed at positions spaced apart from the light sources by a predetermined distance and detect light scattered or reflected from the object. The light sources may emit light of different wavelengths. For example, the light sources may emit light of an infrared wavelength, a green wavelength, a blue wavelength, a red wavelength, a white wavelength, and the like. The light sources may include a light emitting diode (LED), a laser diode (LD), a phosphor, and the like, but are not limited thereto. Further, the light receivers may include a photodiode, a photodiode array, a complementary metal-oxide semiconductor (CMOS) image sensor (CIS), a charge-coupled device (CCD) image sensor, and the like.

The pulse wave sensor 110 may have a single channel including a light source and a light receiver, so as to measure a pulse wave signal at a specific point of the object. Alternatively, the pulse wave sensor 110 may have multiple channels to measure a plurality of pulse wave signals at multiple points of the object. Each of the channels of the pulse wave sensor 110 may be formed in a pre-defined shape such as a circular shape, an oval shape, a fan shape, etc., so that pulse wave signals may be measured at multiple points of the object. Each channel of the pulse wave sensor 110 may include one or more light sources and one or more light receivers. Further, each channel may include two or more light sources to emit light of a plurality of wavelengths. Alternatively, the pulse wave sensor 110 may be configured to measure a plurality of pulse wave signals in a predetermined area of the object. For example, the pulse wave sensor 110 may include one or more light sources, and a light receiver formed as a CIS and disposed at a predetermined distance from the one or more light sources.

A position sensor 120 may obtain sensor position information when the object is in contact with the pulse wave sensor 110, such as position information on the object being in contact with the pulse wave sensor 110. At least some functions of the position sensor 120 may be integrated with the processor 130.

For example, the position sensor 120 may obtain the sensor position information based on object images captured by an external image capturing device. The external image capturing device may be a camera module installed at a fixed location or a camera module mounted in a mobile device such as a smartphone, and the like. For example, once the external image capturing device captures an image of the object being in contact with the pulse wave sensor 110, the position sensor 120 may receive the image of the object through a communication interface mounted in the apparatus 100 for estimating bio-information.

By analyzing relative positions of the pulse wave sensor 110 and the object based on the image of the object, the position sensor 120 may obtain the position of the object, being in contact with the pulse wave sensor 110, as a sensor position. Further, once the external image capturing device, having a function of obtaining a sensor position, obtains sensor position information by capturing an image of the object, the position sensor 120 may receive the sensor position information through the communication interface.

In another example, the position sensor 120 may include a fingerprint sensor for acquiring a fingerprint image of the object in contact with the pulse wave sensor 110. The fingerprint sensor may be disposed at an upper end or a lower end of the pulse wave sensor 110. The position sensor 120 may estimate a sensor position by analyzing a change in a fingerprint pattern based on the fingerprint image of the object. For example, when a finger applies pressure to the pulse wave sensor 110, a contact position of the finger, which is in contact with the pulse wave sensor 110, is pressed against the pulse wave sensor 110 more than as compared to other positions of the finger, such that a distance between ridges or valleys of a fingerprint of the contact position between the finger and the pulse wave sensor 110 is larger than other positions. If a distance between ridges or valleys of the fingerprint at a position of the finger is greater than or equal to a predetermined threshold value when compared to other positions, the position sensor 120 may obtain the position as a sensor position.

In yet another example, when the pulse wave sensor 110 has multiple channels to measure a plurality of pulse wave signals at the same time, the position sensor 120 may obtain a preset measurement position to measure each pulse wave signal as a sensor position.

The processor 130 may be electrically connected to the pulse wave sensor 110, and may control the pulse wave sensor 110 in response to a request for estimating bio-information. The processor 130 may control the pulse wave sensor 110 to obtain pulse wave signals at a plurality of measurement positions of the object. In this case, if the pulse wave sensor 110 has a single channel including one light source and one receiver, the processor 130 may control the pulse wave sensor 110 a plurality of number of times to obtain pulse wave signals at a plurality of positions of the object.

The processor 130 may estimate bio-information based on the plurality of pulse wave signals obtained at the plurality of sensor positions of the object. Further, the processor 130 may obtain final bio-information based on bio-information obtained at each sensor position, each sensor position information, and blood vessel position information of the object.

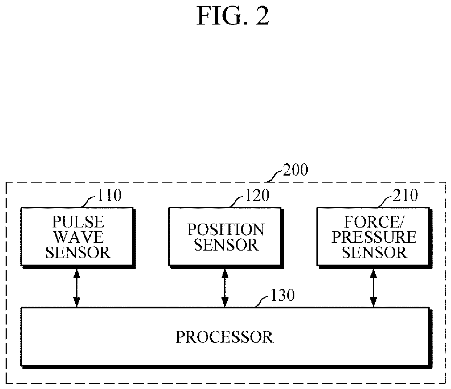

Referring to FIG. 2, an apparatus 200 for estimating bio-information according to another embodiment includes the pulse wave sensor 110, the position sensor 120, the processor 130, and a force/pressure sensor 210. Redundant descriptions of the position sensor 120 and the processor 130 will be omitted.

When a user places an object on the pulse wave sensor 110 and increases or decreases a pressing force/pressure to induce a change in pulse wave amplitude, the force/pressure sensor 210 may measure the force/pressure exerted between the pulse wave sensor 110 and the object. The force/pressure sensor 210 may include a force sensor including a strain gauge, and the like, a force sensor array, an air bladder type pressure sensor, a pressure sensor in combination with a force sensor and an area sensor, and the like.

The processor 130 may estimate bio-information at each sensor position based on the pulse wave signals, obtained at a plurality of sensor positions by the pulse wave sensor 110, and the force/pressure obtained by the force/pressure sensor 210. In this case, once the force/pressure sensor 210 obtains a contact force between the object and the pulse wave sensor 110, the processor 130 may convert the contact force into contact pressure by using a conversion model which defines a correlation between the contact force and the contact pressure. Alternatively, the processor 130 may obtain contact pressure by using the contact force and area information of the pulse wave sensor 110. Furthermore, if the force/pressure sensor 210 is implemented as a force sensor for measuring a contact force and an area sensor for measuring a contact area, the processor 130 may obtain contact pressure based on the contact force, measured by the force sensor, and the contact area measured by the area sensor.

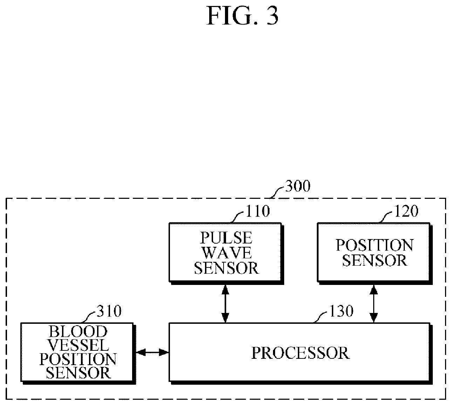

Referring to FIG. 3, an apparatus 300 for estimating bio-information according to yet another embodiment includes the pulse wave sensor 110, the position sensor 120, the processor 130, and a blood vessel position sensor 310. The pulse wave sensor 110, the position sensor 120, and the processor 130 are described above in detail, such that redundant descriptions thereof will be omitted. The force/pressure sensor 210 of FIG. 2 may be included in the apparatus 300 for estimating bio-information according to an embodiment of the present disclosure.

The blood vessel position sensor 310 may obtain blood vessel position information of an object at a time when a user is registered. Alternatively, in response to a user's request for estimating bio-information, the blood vessel position sensor 310 may check whether there is blood vessel position information of the user's object or whether it is time to calibrate the information; and if there is no blood vessel position information of the object or it is time to calibrate the information, the blood vessel position obtainer 310 may obtain blood vessel position information of the object from the user. At least some functions of the blood vessel position sensor 310 may be integrated with the processor 130. Examples of obtaining blood vessel positions by the blood vessel position sensor 310 will be described below, but the present disclosure is not limited to these examples.

For example, the blood vessel position sensor 310 may directly receive input of blood vessel position information from a user. In this case, the blood vessel position sensor 310 may display an image of the object on a display, and may provide an interface for the user to directly designate a blood vessel position on the object image by using an input means (e.g., finger, touch pen, etc.).

In another example, the blood vessel position sensor 310 may receive images, which are captured by an external image capturing device for capturing optical images, ultrasonic images, magnetic resonance imaging (MRI) images, photoacoustic images, etc., through the communication interface, and may obtain blood vessel position information of the object by analyzing the received images. Alternatively, if the external image capturing device analyzes a blood vessel position while capturing the images, the blood vessel position sensor 310 may receive the blood vessel position information of the obj ect through the communication interface.

In yet another example, the blood vessel position sensor 310 may include, for example, an ultrasonic sensor which transmits an ultrasonic wave to the object, and receives a reflection wave from the object. The blood vessel position sensor 310 may obtain blood vessel position information based on ultrasonic images obtained by the ultrasonic sensor.

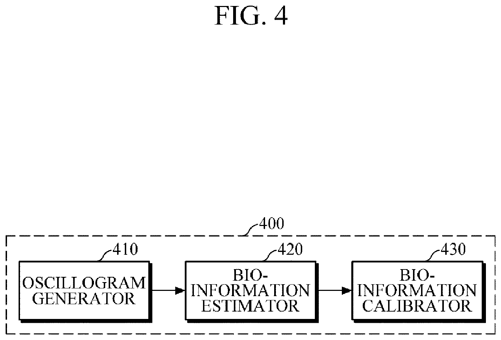

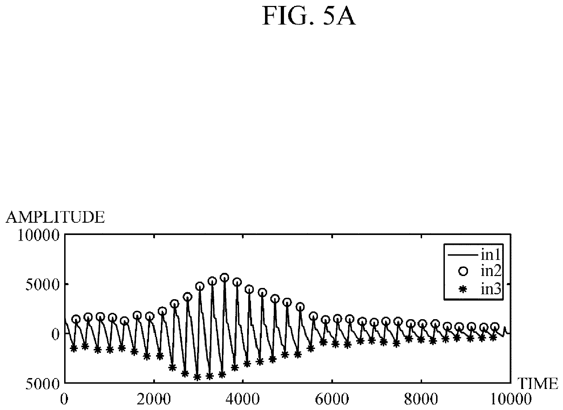

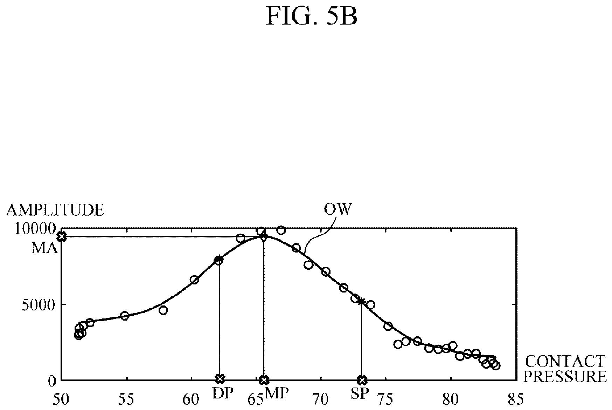

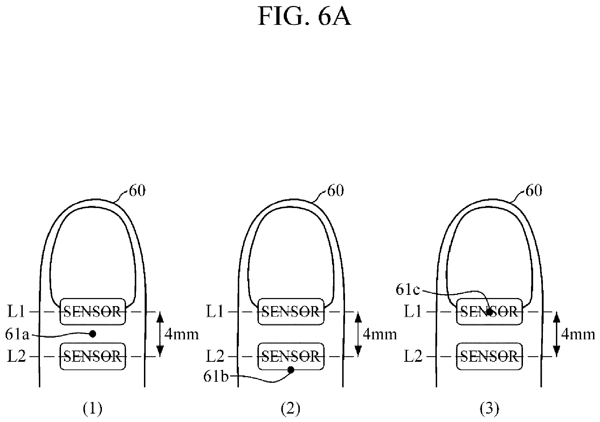

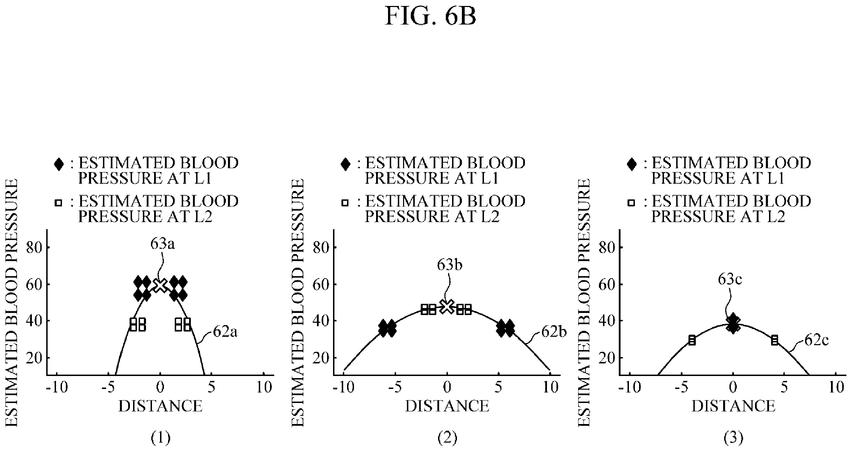

FIG. 4 is an example of a configuration of a processor of the apparatus for estimating bio-information illustrated in FIGS. 1 to 3. FIGS. 5A and 5B are diagrams explaining an example of estimating blood pressure using oscillometry. FIGS. 6A and 6B are diagrams explaining an example of calibrating blood pressure based on blood vessel positions of an object.

Referring to FIG. 4, a processor 400 according to an embodiment of the present disclosure includes an oscillogram generator 410, a bio-information estimator 420, and a bio-information calibrator 430.

The oscillogram generator 410 may generate an oscillogram for each sensor position based on pulse wave signals, measured at each sensor position by the pulse wave sensor 110, and contact pressure.

For example, referring to FIGS. 5A and 5B, the oscillogram generator 410 may extract a peak-to-peak point of the pulse wave signal waveform by subtracting a negative (-) amplitude value in3 from a positive (+) amplitude value in2 of a waveform envelope in1 at each measurement time of the pulse wave signal, and may obtain the oscillogram (OW) by plotting the peak-to-peak amplitude at each measurement time against the contact pressure value at a corresponding time and by performing polynomial curve fitting.

The bio-information estimator 420 may extract characteristic points from the oscillogram for each sensor position, and may estimate bio-information for each sensor position by using the extracted characteristic points. For example, the bio-information estimator 420 may extract, as characteristic points, a contact pressure value MP at a point corresponding to a maximum amplitude value, contact pressure values DP and SP at points corresponding to amplitude values having a preset ratio (e.g., 0.5 to 0.7) to a maximum amplitude value MA, and the like.

The bio-information estimator 420 may determine, for example, the contact pressure value MP at a point, corresponding to the maximum amplitude value, as mean arterial pressure (MAP); and may determine contact pressure values DP and SP at the left and right points, corresponding to amplitude values having a preset ratio to the maximum amplitude value, as diastolic blood pressure (DBP) and systolic blood pressure (SBP), respectively. Alternatively, the bio-information estimator 420 may independently estimate the MAP, DBP, and SBP by applying each of the extracted contact pressure values MP, DP, and SP to a pre-defined blood pressure estimation model. In this case, the blood pressure estimation model may be expressed in the form of various linear or non-linear combination functions, such as addition, subtraction, division, multiplication, logarithmic value, regression equation, and the like, with no particular limitation.

The bio-information calibrator 430 may obtain final bio-information based on the bio-information generated for each sensor position by the bio-information estimator 420. For example, the bio-information calibrator 430 may generate a calibration graph by plotting estimated bio-information values for each sensor position against a relative distance of each sensor position from the blood vessel position of the object, and by fitting the curve. Further, the bio-information calibrator 430 may obtain a final estimated bio-information value based on the generated calibration graph.

FIG. 6A illustrates, in (1), an example in which a first sensor position L1 is a tip of a fingernail, a second sensor position L2 is located at a distance of 4 mm from the first sensor position L1, and a blood vessel 61a of a finger 60 is located between the first sensor position L1 and the second sensor position L2. In this case, a relative distance of both the first sensor position L1 and the second sensor position L2 from the blood vessel position 61a is 2 mm.

Referring to (1) of FIG. 6B, the bio-information calibrator 430 may locate the blood vessel position 61a at "0" on the X axis, and then may plot an estimated blood pressure value of the first sensor position L1 at distances of "-2" and "+2" from the distance of "0" on the X axis, and may plot an estimated blood pressure value of the second sensor position L2 at distances of "+2" and"-2" on the X axis. Then, upon plotting the estimated blood pressure values of the first and second sensor positions L1 and L2, the bio-information calibrator 430 may perform curve fitting to generate a calibration graph 62a of, for example, a quadratic function. In this case, various known curve fitting techniques may be used to perform the curve fitting. Upon generating the calibration graph 62a, the bio-information estimator 420 may obtain a blood pressure value at a blood vessel position, i.e., a point 63a corresponding to "0" on the X axis in the calibration graph 62a, as a final blood pressure value.

FIG. 6a illustrates, in (2), an example in which a blood vessel 61b of the finger 60 is located at a distance of 6 mm from the first sensor position L1 and at a distance of 2 mm from the second sensor position L2, in which case a relative distance of the first sensor position L1 from the blood vessel position 61a is 6 mm, and a relative distance of the second sensor position L2 therefrom is 2 mm. As illustrated in (2) of FIG. 6B, the bio-information calibrator 430 may locate the blood vessel position 61b at "0" on the X axis, and then may plot an estimated blood pressure value of the first sensor position L1 at distances of "-6" and "+6" on the X axis, and may plot an estimated blood pressure value of the second sensor position L2 at distances of "+2" and"-2" on the X axis. Then, the bio-information calibrator 430 may perform curve fitting to generate a calibration graph 62b showing a relatively smooth curve. In this case, the bio-information estimator 420 may obtain a blood pressure value at a blood vessel position, i.e., a point 63b corresponding to "0" on the X axis in the calibration graph 62b, as a final blood pressure value.

FIG. 6A illustrates, in (3), an example in which a blood vessel 61c of the finger 60 is superimposed on the first sensor position L1, and a relative distance of the first sensor position L1 from the blood vessel position 61a is 0 mm and a relative distance of the second sensor position L2 therefrom is 4 mm. As illustrated in (3) of FIG. 6B, the bio-information calibrator 430 may generate a calibration graph 62c by plotting an estimated blood pressure value of the first sensor position L1 at "0" on the X axis and an estimated blood pressure value of the second sensor position L2 at distances of "+4" and"-4" on the X axis, and by performing curve fitting. In this case, the bio-information estimator 420 may obtain a blood pressure value at a blood vessel position, i.e., a point 63c corresponding to "0" on the X axis in the calibration graph 62c, as a final blood pressure value.

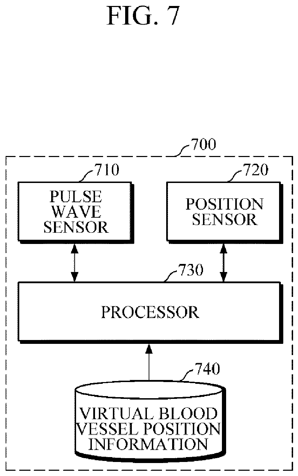

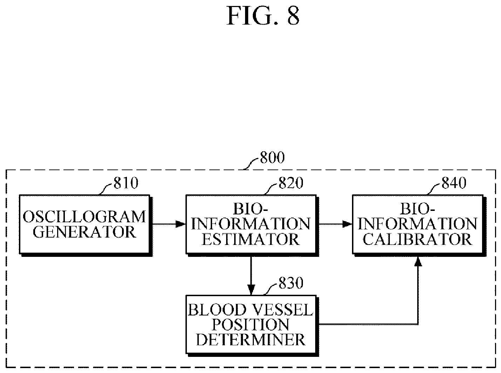

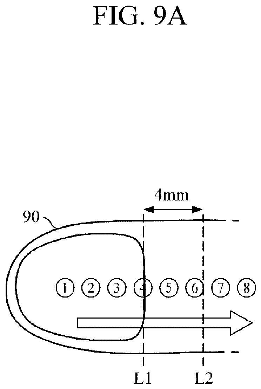

FIG. 7 is a block diagram illustrating an apparatus for estimating bio-information according to another embodiment of the present disclosure. FIG. 8 is a diagram illustrating an example of a configuration of a processor of FIG. 7. FIGS. 9A to 9C are diagrams explaining an example of calibrating blood pressure based on a virtual blood vessel position.

Referring to FIG. 7, the apparatus 700 for estimating bio-information according to another embodiment includes a pulse wave sensor 710, a position sensor 720, a processor 730, and a virtual blood vessel position information 740. Embodiments of the pulse wave sensor 710 and the position sensor 720 are described in detail above.

Once pulse wave signals are obtained at a plurality of positions of an object, the processor 730 may obtain final bio-information based on the pre-defined virtual blood vessel position information 740.

The virtual blood vessel position information 740 may be obtained from a plurality of users by an external device or the apparatus 700 for estimating bio-information. The virtual blood vessel position information 740 may be pre-stored in a storage of the apparatus 700 for estimating bio-information. The virtual blood vessel position information 740 may include a plurality of virtual blood vessel positions for each object, in which case one virtual blood vessel position may be set for each of a plurality of groups which are classified according to predetermined criteria.

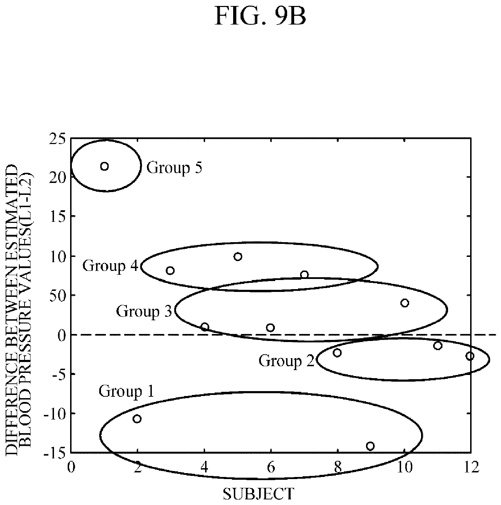

For example, referring to FIG. 9A, by measuring pulse wave signals at each of the first sensor position L1 and the second sensor position L2 of a user's finger 90, and by moving virtual blood vessel positions from position 1 to position 8, a calibration graph may be generated for each of the virtual blood vessel positions and a final blood pressure value may be estimated, as described above. By using data remaining after excluding abnormal data based on the generated calibration graph or the estimated final blood pressure value, a plurality of groups may be classified as illustrated in FIG. 9B. For example, as illustrated in FIG. 9B, a plurality of groups may be classified based on differences between the estimated blood pressure at the first sensor position L1 and the estimated blood pressure at the second sensor position L2. However, the example of classifying the groups is not limited thereto, and the groups may be classified by various linear/non-linear combinations, including a ratio between the estimated blood pressure at the first sensor position L1 and the estimated blood pressure at the second sensor position L2.

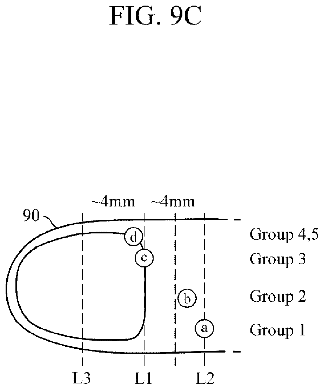

Referring to FIG. 9C, a virtual blood vessel position may be defined for each group. For example, in Group 1, the estimated pressure value of the second sensor position L2 is much greater than the estimated pressure value of the first sensor position L1, such that a position (a) superimposed on the second sensor position L2 may be defined as the virtual blood vessel position for Group 1. In this manner, a position (b) of the object may be defined as the virtual blood vessel position for Group 2, a position (c) may be defined as the virtual blood vessel position for Group 3, and a position (d) may be defined as the virtual blood vessel position for Groups 4 and 5.

Referring to FIG. 8, a processor 800 according to an embodiment includes an oscillogram generator 810, a bio-information estimator 820, a blood vessel position determiner 830, and a bio-information calibrator 840.

The oscillogram generator 810 may generate an oscillogram for each sensor position based on pulse wave signals, measured by the pulse wave sensor 710 at each sensor position, and contact pressure.

The bio-information estimator 820 may extract characteristic points from the oscillogram OW for each sensor position, and may estimate bio-information for each sensor position by using the extracted characteristic points.

The blood vessel position determiner 830 may determine an optimal blood vessel position among a plurality of virtual blood vessel positions, based on the virtual blood vessel position information 740 and the bio-information for each sensor position. For example, the blood vessel position determiner 830 may calculate, as pre-defined group classification criteria, a difference value between the estimated blood pressure of the first sensor position L1 and the estimated blood pressure of the second sensor position L2, and may determine a group to which the calculated difference value belongs. Further, based on the virtual blood vessel position information 740, the blood vessel position determiner 830 may determine a virtual blood vessel position, corresponding to the determined group, as an optimal (or improved) blood vessel position of a user's object.

Once the blood vessel position determiner 830 determines the optimal blood vessel position of the object, the bio-information calibrator 840 may generate a calibration graph based on a relative distance between the optimal blood vessel position and each of the first and second sensor positions L1 and L2 as described above, and may obtain an estimated value, corresponding to the blood vessel position in the calibration graph, as final bio-information.

According to this embodiment, bio-information may be estimated accurately even when accurate blood vessel information may not be obtained from a user's object, and the apparatus may be manufactured in a compact size as there is no need for a separate sensor.

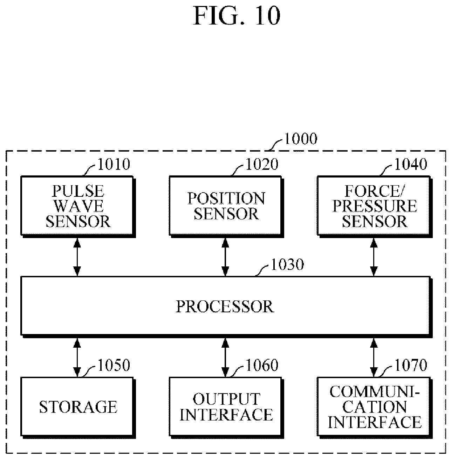

FIG. 10 is a block diagram illustrating an apparatus for estimating bio-information according to yet another embodiment of the present disclosure.

Referring to FIG. 10, an apparatus 1000 for estimating bio-information according to another embodiment includes a pulse wave sensor 1010, a position sensor 1020, a force/pressure sensor 1040, a processor 1030, a storage 1050, an output interface 1060, and a communication interface 1070. Various embodiments of the pulse wave sensor 1010, the position sensor 1020, the force/pressure sensor 1040, and the processor 1030 are described in detail above, such that redundant description thereof will be omitted.

The storage 1050 may store a variety of information required for estimating bio-information. For example, the storage 1050 may store pulse wave signals measured by the pulse wave sensor 1010, object images, fingerprint images, and sensor position information which are obtained by the position sensor 1020, force/pressure values obtained by the force/pressure sensor 1040, and the like. Further, the storage 1050 may store processing results of the processor 1030, such as an estimated bio-information value for each sensor position, a calibration graph, a final estimated bio-information value, and the like. In addition, the storage 1050 may store blood vessel position information of a user's object, and user characteristic information such as a user's age, gender, health condition, and the like. Moreover, the storage 1050 may store virtual blood vessel position information and the like. However, the information is not limited thereto.

The storage 1050 may include at least one storage medium of a flash memory type memory, a hard disk type memory, a multimedia card micro type memory, a card type memory (e.g., a secure digital (SD) memory, an extreme digital (XD) memory, etc.), a Random Access Memory (RAM), a Static Random Access Memory (SRAM), a Read Only Memory (ROM), an Electrically Erasable Programmable Read Only Memory (EEPROM), a Programmable Read Only Memory (PROM), a magnetic memory, a magnetic disk, and an optical disk, and the like, but is not limited thereto.

The output interface 1060 may output the pulse wave signals measured by the pulse wave sensor 1010, the object images, the fingerprint images, and the sensor position information which are obtained by the position sensor 1020, the force/pressure values obtained by the force/pressure sensor 1040, and/or the processing results of the processor 1030. In this case, along with the visual display of the information on a display, the output interface 1060 may provide the information by a non-visual method using a speaker, a haptic device, and the like.

For example, the output interface 1060 may output the measured pulse wave signal in the form of graphs. Further, the output interface 1060 may visually display an estimated blood pressure value of a user by using various visual methods, such as by changing color, line thickness, font, and the like, based on whether the estimated blood pressure value falls within or outside a normal range. Alternatively, upon comparing the estimated blood pressure value with a previous estimation history, if it is determined that the estimated blood pressure value is abnormal, the output interface 1060 may provide a warning message and the like, as well as guide information on a user's action such as food information that the user should be careful about, related hospital information, and the like. The output interface 1060 may guide a user on a contact position of an object based on the position information obtained by the position sensor 1020. In addition, based on the force/pressure obtained by the force/pressure sensor 1040, the output interface 1060 may guide force/pressure to be applied by the object to the pulse wave sensor 1010.

The communication interface 1070 may communicate with an external device by using wired or wireless communication techniques under the control of the processor 1030, and may transmit and receive various data to and from the external device. For example, the communication interface 1070 may transmit a bio-information estimation result to the external device, and may receive a variety of reference information required for estimating bio-information from the external device. In this case, the external device may include an information processing device, such as a cuff-type blood pressure measuring device, a smartphone, a tablet PC, a desktop computer, a laptop computer, and the like.

In this case, examples of the communication techniques may include Bluetooth communication, Bluetooth Low Energy (BLE) communication, Near Field Communication (NFC), wireless local area network (WLAN) communication, Zigbee communication, Infrared Data Association (IrDA) communication, wireless fidelity (Wi-Fi) Direct (WFD) communication, Ultra-Wideband (UWB) communication, Ant+ communication, Wi-Fi communication, Radio Frequency Identification (RFID) communication, 3G communication, 4G communication, 5G communication, and the like. However, this is merely exemplary and is not intended to be limiting.

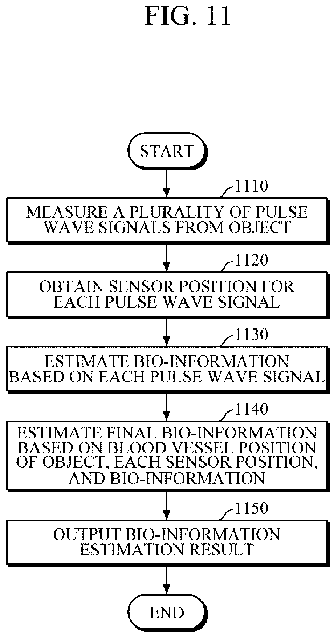

FIG. 11 is a flowchart illustrating a method of estimating bio-information according to an embodiment of the present disclosure. The method of FIG. 11 is an example of a method of estimating bio-information which is performed by the aforementioned apparatuses 100, 200, 300, and 1000 for estimating bio-information, which are described above in detail, and thus will be briefly described below.

The apparatuses 100, 200, 300, and 1000 for estimating bio-information may measure a plurality of pulse wave signals at a plurality of positions of a user's object by using a pulse wave sensor in operation 1110. While the user places the object on the pulse wave sensor, the user may change contact pressure to induce a change in pulse wave amplitude. In this case, a change in contact force/contact pressure may be obtained by a force/pressure sensor.

Then, while the pulse wave signals are obtained at the plurality of positions of the object, the apparatuses 100, 200, 300, and 1000 for estimating bio-information may obtain a position of the pulse wave sensor on the object in operation 1120. For example, when the object is in contact with the pulse wave sensor, sensor position information may be obtained based on an image of the object, captured by an external image capturing device, or a fingerprint image obtained by fingerprint sensor mounted in the apparatuses.

Subsequently, the apparatuses 100, 200, 300, and 1000 for estimating bio-information may estimate bio-information at each sensor position based on the pulse wave signals obtained at each sensor position in operation 1130. For example, the apparatuses 100, 200, 300, and 1000 for estimating bio-information may generate an oscillogram based on the contact force/contact pressure, obtained while the pulse wave signals are measured, and the pulse wave signals of each sensor, and may estimate bio-information by using the generated oscillogram.

Next, the apparatuses 100, 200, 300, and 1000 for estimating bio-information may estimate final bio-information based on blood vessel position information of the object, the sensor position information, and bio-information at each sensor position in operation 1140. For example, the apparatuses 100, 200, 300, and 1000 for estimating bio-information may generate a calibration graph by calculating a relative distance of each sensor position from the blood vessel position, plotting the bio-information of each sensor position against the relative distance, and performing curve fitting. Further, upon generating the calibration graph, the apparatuses 100, 200, 300, and 1000 for estimating bio-information may obtain bio-information at a point, corresponding to the blood vessel position in the calibration graph, as the final bio-information.

Then, the apparatuses 100, 200, 300, and 1000 for estimating bio-information may output a bio-information estimation result in operation 1150. The apparatuses 100, 200, and 300 for estimating bio-information may provide a user with information, such as the estimated bio-information values, a warning, measurements, a bio-information estimation history, etc., by using a display, a speaker, a haptic device, and the like.

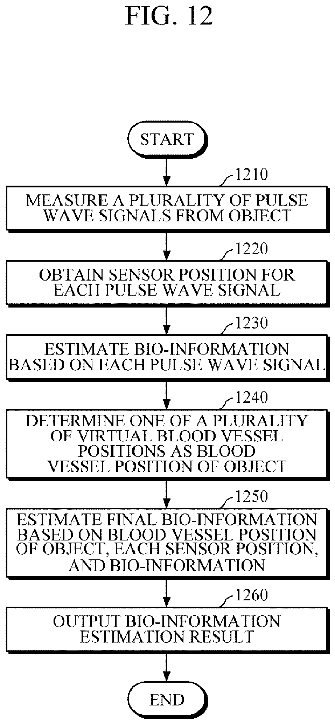

FIG. 12 is a flowchart illustrating a method of estimating bio-information according to another embodiment of the present disclosure. The method of FIG. 12 is an example of a method of estimating bio-information which is performed by the aforementioned apparatuses 700 and 1000 for estimating bio-information, which is described above in detail, and thus will be briefly described below.

The apparatuses 700 and 1000 for estimating bio-information may measure a plurality of pulse wave signals at a plurality of positions of a user's object by using a pulse wave sensor in operation 1210, and may obtain a position of the pulse wave sensor on the object in operation 1220.

Then, the apparatuses 700 and 1000 for estimating bio-information may estimate bio-information at each sensor position based on the pulse wave signals obtained at each sensor position in operation 1230.

Subsequently, the apparatuses 700 and 1000 for estimating bio-information may determine one of a plurality of virtual blood vessel positions as an optimal (or improved) blood vessel position of the object in operation 1240. For example, the apparatus 700 for estimating bio-information may determine a group to which a difference value between an estimated blood pressure value at the first sensor position and an estimated blood pressure value at the second sensor position belongs, and may determine a virtual blood vessel position of the determined group as the optimal blood vessel position of the user's object.

Next, the apparatuses 700 and 1000 for estimating bio-information may estimate final bio-information based on the determined optimal vessel position information of the object, the sensor position information, the bio-information at each sensor position in operation 1250, and may output a bio-information estimation result in operation 1260.

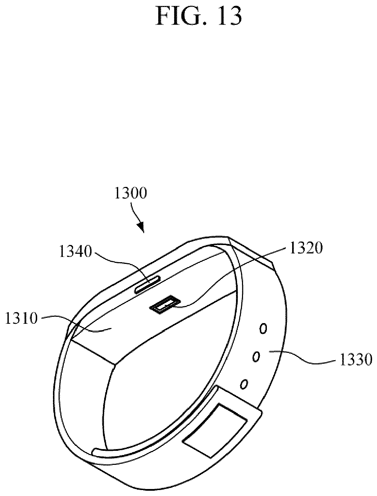

FIG. 13 is a diagram illustrating an example of a wearable device. Various embodiments of the aforementioned apparatuses for estimating bio-information may be mounted in the wearable device.

Referring to FIG. 13, the wearable device 1300 includes a main body 1310 and a strap 1330.

The strap 1330, which is connected to both ends of the main body 1310, may be flexible so as to be bent around a user's wrist. The strap 1330 may be composed of a first strap and a second strap which are separated from each other. Respective ends of the first strap and the second strap are connected to the main body 1310, and the other ends thereof may be connected to each other via a connecting means. In this case, the connecting means may be formed as magnetic connection, Velcro connection, pin connection, and the like, but is not limited thereto. Further, the strap 1330 is not limited thereto, and may be integrally formed as a non-detachable band.

In this case, air may be injected into the strap 1330, or the strap 1330 may be provided with an air bladder, so that the strap 1330 may have elasticity according to a change in pressure applied to the wrist, and may transmit the change in pressure of the wrist to the main body 1310.

A battery may be embedded in the main body 1310 or the strap 1330 to supply power to the wearable device 1300.

Furthermore, the main body 1310 may include a sensor part 1320 mounted on one side thereof. The sensor part 1320 may include a pulse wave sensor for measuring pulse wave signals. The pulse wave sensor may include a light source for emitting light onto skin of a wrist or a finger, a light receiver such as a CIS optical sensor for detecting light scattered or reflected from the wrist or the finger, a photodiode, and the like. The pulse wave sensor may have multiple channels for measuring pulse wave signals at multiple points of the wrist or the finger, and each of the channels may include a light source and a light receiver, and may include a plurality of light sources for emitting light of different wavelengths. In addition, the sensor part 1320 may further include a force/pressure sensor for measuring force/pressure between the wrist or finger and the sensor part 1320. Moreover, the sensor part 1320 may further include a fingerprint sensor, an ultrasonic sensor, and the like, which may be stacked on top of each other.

A processor may be mounted in the main body 1310. The processor may be electrically connected to modules mounted in the wearable device 1300. Based on the pulse wave signals and the contact force/pressure, which are measured by the sensor part 1320 at a plurality of measurement positions of the object, the processor may estimate blood pressure at each measurement position using oscillometry based on the pulse wave signals, measured at a plurality of measurement positions of the object, and the contact force/pressure, and may estimate final blood pressure by calibrating the estimated blood pressure at each measurement position based on the blood vessel position of the object. In this case, the blood vessel position of the object may be an actual blood vessel position of the object which is obtained based on user input, an ultrasonic image, an MRI image, an optical image, and the like. Alternatively, if it is difficult to obtain an actual blood vessel position of the object, the blood vessel position may be an optimal blood vessel position which is selected according to predetermined criteria from among pre-defined virtual blood vessel positions for a plurality of users.

Further, the main body 1310 may include a storage which stores reference information for estimating blood pressure and performing various functions of the wearable device 1300, and information processed by various modules thereof.

In addition, the main body 1310 may include a manipulator 1340 which is provided on one side surface of the main body 1310, and receives a user's control command and transmits the received control command to the processor. The manipulator 1340 may have a power button to input a command to turn on/off the wearable device 1300.

Further, a display for outputting information to a user may be mounted on a front surface of the main body 1310. The display may have a touch screen for receiving touch input. The display may receive a user's touch input and transmit the touch input to the processor, and may display processing results of the processor.

Moreover, the main body 1310 may include a communication interface for communication with an external device. The communication interface may transmit a blood pressure estimation result to the external device, such as a user's smartphone.

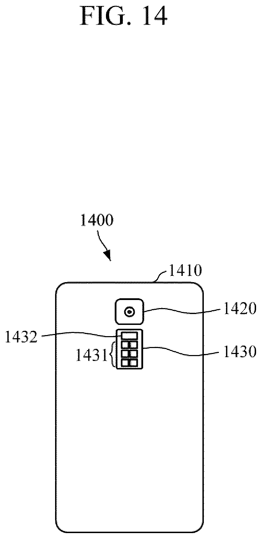

FIG. 14 is a diagram illustrating an example of a smart device. In this case, the smart device may include a smartphone, a tablet PC, and the like. The smart device may include various embodiments of the aforementioned apparatuses for estimating bio-information.

Referring to FIG. 14, the smart device 1400 includes a main body 1410 and a pulse wave sensor 1430 mounted on one surface of the main body 1410. For example, the pulse wave sensor 1430 may include one or more light sources 1432 disposed at predetermined positions thereof. The one or more light sources 1432 may emit light of different wavelengths. In addition, a plurality of light receivers 1431 may be disposed at predetermined distances from the light sources 1432. However, this is merely an example, and the pulse wave sensor 1430 may have various shapes as described above. Further, a force/pressure sensor for measuring a contact force/pressure of a finger may be mounted in the main body 1410 at a lower end of the pulse wave sensor 1430.

Moreover, a display may be mounted on a front surface of the main body 1410. The display may visually output a blood pressure estimation result, a health condition evaluation result, and the like. The display may include a touch screen, and may receive information input through the touch screen and transmit the information to a processor.

The main body 1410 may include an image sensor 1420 as illustrated in FIG. 10. The image sensor 1420 may capture various images, and may obtain, for example, a fingerprint image of a finger being in contact with the pulse wave sensor 1430. In addition, when an image sensor based on the CIS technology is mounted in the light receiver 1431 of the pulse wave sensor 1430, the image sensor 1420 may be omitted.

The processor may estimate blood pressure based on the blood vessel position of the object and sensor position information obtained when the object is in contact with the sensor, as described above.

The embodiments of the present disclosure can be implemented by computer-readable code written on a non-transitory computer-readable medium that is executed by a processor. The non-transitory computer-readable medium may be any type of recording device in which data is stored in a computer-readable manner.

Examples of the non-transitory computer-readable medium include a ROM, a RAM, a CD-ROM, a magnetic tape, a floppy disc, an optical data storage, and a carrier wave (e.g., data transmission through the Internet). The non-transitory computer-readable medium can be distributed over a plurality of computer systems connected to a network so that computer-readable code is written thereto and executed therefrom in a decentralized manner. Functional programs, code, and code segments for implementing the embodiments of the present disclosure can be deduced by programmers of ordinary skill in the art to which the present disclosure pertains.

## Claims

1. An apparatus (100) for estimating bio-information of a user, the apparatus (100) comprising:
a pulse wave sensor (110) configured to measure (1110) a plurality of pulse wave signals from an object of the user;
a position sensor (120) configured to obtain (1120) sensor position information identifying a sensor position on the object for each of the plurality of pulse wave signals, based on the pulse wave sensor (110) measuring each of the plurality of pulse wave signals; and
a processor (130) configured to:
estimate (1130) first bio-information at each sensor position based on each of the plurality of pulse wave signals;
generate a calibration graph (62) by plotting the estimated first bio-information values at each sensor position against a relative distance of each sensor position from a blood vessel position (61) of the object;
based on performing curve fitting, obtain (1140) a second estimated bio-information value based on the calibration graph (62); and
obtain (1150) a bio-information value at a point, corresponding to the blood vessel position (61) of the object in the calibration graph (62), as the second bio-information.

2. The apparatus (100) of claim 1, wherein based on the object being in contact with the pulse wave sensor (110), the position sensor (120) is further configured to obtain (1120) the sensor position information based on an image of the object which is captured by an external capturing device or based on pre-defined measurement position information of the pulse wave sensor (110).

3. The apparatus (100) of claim 1 or 2, wherein the position sensor (120) comprises a fingerprint sensor configured to obtain a fingerprint image, and wherein the position sensor (120) is further configured to obtain (1120) the sensor position information based on the fingerprint image obtained by the fingerprint sensor based on the object being in contact with the pulse wave sensor (110), or
further comprising a blood vessel position sensor (310) configured to obtain the blood vessel position information of the object based on at least one of an optical image, an ultrasonic image, a magnetic resonance imaging, MRI, image, and a photoacoustic image, of the object which are obtained by an external device, or
further comprising a blood vessel position sensor (310) which includes an ultrasonic sensor configured to transmit an ultrasonic wave to the object and receive a signal reflected from the object, and obtain the blood vessel position information of the object based on an ultrasonic image obtained by the ultrasonic sensor.

4. The apparatus (100) of one of claims 1 to 3, further comprising:
a force sensor (210) configured to measure a force applied by the object to the pulse wave sensor (110); or
a pressure sensor (210) configured to measure a pressure applied by the object to the pulse wave sensor (110).

5. The apparatus (100) of claim 4, wherein the processor (130) is further configured to:
generate an oscillogram based on each of the plurality of pulse wave signals and the force measured by the force sensor (210) or the pressure measured by the pressure sensor (210); and
estimate the first bio-information at each sensor position by using the oscillogram.

6. The apparatus (100) of one of claims 1 to 5, wherein the bio-information comprises one or more of blood pressure, vascular age, arterial stiffness, aortic pressure waveform, vascular compliance, stress index, fatigue level, skin age, and skin elasticity.

7. The apparatus (100) of one of claims 1 to 6, wherein the processor (130) is further configured to determine (1240) one of a plurality of virtual blood vessel positions as a blood vessel position of the object based on the first bio-information at each sensor position.

8. The apparatus (100) of claim 7, wherein based on a difference between first bio-information values at each sensor position, the processor (130) is further configured to determine (1240) the one of the plurality of virtual blood vessel positions as the blood vessel position (61) of the object.

9. The apparatus (100) of claim 8, wherein a virtual blood vessel position is set for each of a plurality of groups which are pre-classified based on the difference between the first bio-information values at each sensor position obtained from a plurality of users, or
wherein the processor (130) is further configured to:
determine a group, to which the difference belongs, among the plurality of groups; and
determine a virtual blood vessel position, pre-defined for the determined group, as the blood vessel position (61) of the object.

10. A method of estimating bio-information of a user, the method comprising:
measuring (1110) by a pulse wave sensor (110), a plurality of pulse wave signals from an object;
obtaining (1120) by a position sensor (120), sensor position information identifying a sensor position on the object for each of the plurality of pulse wave signals, based on the pulse wave sensor (110) measuring each of the plurality of pulse wave signals;
estimating (1130) by a processor (130), first bio-information at each sensor position based on each of the plurality of pulse wave signals; and
estimating (1140) by the processor, second bio-information based on a blood vessel position of the object each sensor position, and the first bio-information at each sensor position, wherein the estimating (1140) of the second bio-information comprises generating a calibration graph (62) by plotting first estimated bio-information values at each sensor position against a relative distance of each sensor position from the blood vessel position of the object, and by performing curve fitting, obtaining (1150) the second estimated bio-information value based on the calibration graph (62).

11. The method of claim 10 further comprising determining (1240) one of a plurality of virtual blood vessel positions as a blood vessel position of the object, based on the first bio-information at each sensor position.

12. The method of claim 10 or 11 adapted to operate the apparatus (100) for estimating bio-information of the user according to one of claims 1 to 9.

## Patentansprüche

1. Vorrichtung (100) zum Schätzen von Bio-Informationen eines Benutzers, wobei die Vorrichtung (100) umfasst:
einen Impulswellensensor (110), der konfiguriert ist, um eine Vielzahl von Impulswellensignalen von einem Objekt des Benutzers zu messen (1110);
einen Positionssensor (120), der konfiguriert ist, um Sensorpositionsinformationen zu beziehen (1120), die eine Sensorposition an dem Objekt für jedes aus der Vielzahl von Impulswellensignalen auf der Basis des Impulswellensensors (110) identifizieren, der jedes aus der Vielzahl von Impulswellensignalen misst; und
einen Prozessor (130), der konfiguriert ist zum:
Schätzen (1130) von ersten Bio-Informationen an jeder Sensorposition auf der Basis von jedem aus der Vielzahl von Impulswellensignalen;
Generieren einer Kalibrierkurve (62) durch Plotten der geschätzten ersten Bio-Informationswerte an jeder Sensorposition gegenüber einem relativen Abstand jeder Sensorposition von einer Blutgefäßposition (61) des Objekts;
auf der Basis der Durchführung einer Kurvenanpassung Beziehen (1140) eines zweiten geschätzten Bio-Informationswerts auf der Basis der Kalibrierkurve (62) und
Beziehen (1150) eines Bio-Informationswerts an einem Punkt, der der Blutgefäßposition (61) des Objekts in der Kalibrierkurve (62) entspricht, als die zweiten Bio-Informationen.

2. Vorrichtung (100) nach Anspruch 1, wobei auf der Basis des mit dem Impulswellensensor (110) in Kontakt befindlichen Objekts der Positionssensor (120) des Weiteren konfiguriert ist, um die Sensorpositionsinformationen auf der Basis eines Bildes des Objekts, das von einer externen Aufnahmevorrichtung aufgenommen wurde, oder auf der Basis von vordefinierten Messpositionsinformationen des Impulswellensensors (110) zu beziehen (1120).

3. Vorrichtung (100) nach Anspruch 1 oder 2, wobei der Positionssensor (120) einen Fingerabdrucksensor umfasst, der konfiguriert ist, um ein Fingerabdruckbild zu beziehen, und wobei der Positionssensor (120) des Weiteren konfiguriert ist, um die Sensorpositionsinformationen auf der Basis des Fingerabdruckbildes zu beziehen (1120), das von dem Fingerabdrucksensor auf der Basis des mit dem Impulswellensensor (110) in Kontakt stehenden Objekts bezogen wird, oder
des Weiteren einen Blutgefäßpositionssensor (310) umfasst, der konfiguriert ist, um die Blutgefäßpositionsinformationen des Objekts auf der Basis von wenigstens einem optischen Bild, einem Ultraschallbild, einem Magnetresonanzbild (Magnetic Resonance Imaging - MRI) oder einem von einer externen Vorrichtung bezogenen photoakustischen Bild des Objekts zu beziehen, oder
des Weiteren einen Blutgefäßpositionssensor (310) umfasst, der einen Ultraschallsensor aufweist, der konfiguriert ist, um eine Ultraschallwelle an das Objekt zu senden und ein von dem Objekt reflektiertes Signal zu empfangen und die Blutgefäßpositionsinformationen des Objekts auf der Basis eines von dem Ultraschallsensor bezogenen Ultraschallbildes zu beziehen.

4. Vorrichtung (100) nach einem der Ansprüche 1 bis 3, die des Weiteren umfasst:
einen Kraftsensor (210), der konfiguriert ist, um eine von dem Objekt auf den Impulswellensensor (110) ausgeübte Kraft zu messen; oder
einen Drucksensor (210), der konfiguriert ist, um einen von dem Objekt auf den Impulswellensensor (110) ausgeübten Druck zu messen.

5. Vorrichtung (100) nach Anspruch 4, wobei der Prozessor (130) des Weiteren konfiguriert ist zum:
Generieren eines Oszillogramms auf der Basis von jedem aus der Vielzahl von Impulswellensignalen und der von dem Kraftsensor (210) gemessenen Kraft oder des von dem Drucksensor (210) gemessenen Drucks und
Schätzen der ersten Bio-Informationen an jeder Sensorposition mittels des Oszillogramms.

6. Vorrichtung (100) nach einem der Ansprüche 1 bis 5, wobei die Bio-Informationen Blutdruck, Gefäßalter, arterielle Steifigkeit, Aortendruckwellenform, Gefäßdehnbarkeit, Stressindex, Ermüdungsgrad, Hautalter und/oder Hautelastizität umfassen.

7. Vorrichtung (100) nach einem der Ansprüche 1 bis 6, wobei der Prozessor (130) des Weiteren konfiguriert ist, um eine aus einer Vielzahl von virtuellen Blutgefäßpositionen als eine Blutgefäßposition des Objekts auf der Basis der ersten Bio-Informationen an jeder Sensorposition zu bestimmen (1240).

8. Vorrichtung (100) nach Anspruch 7, wobei der Prozessor (130) des Weiteren konfiguriert ist, um auf der Basis einer Differenz zwischen ersten Bio-Informationswerten an jeder Sensorposition die eine aus der Vielzahl von virtuellen Blutgefäßpositionen als die Blutgefäßposition (61) des Objekts zu bestimmen (1240).

9. Vorrichtung (100) nach Anspruch 8, wobei eine virtuelle Blutgefäßposition für jede aus einer Vielzahl von Gruppen eingestellt wird, die auf der Basis der Differenz zwischen den ersten Bio-Informationswerten an jeder Sensorposition vorklassifiziert werden, die von einer Vielzahl von Benutzern bezogen wurden, oder
wobei der Prozessor (130) des Weiteren konfiguriert ist zum:
Bestimmen einer Gruppe, zu der die Differenz gehört, unter der Vielzahl von Gruppen; und
Bestimmen einer virtuellen Blutgefäßposition, die für die bestimmte Gruppe vordefiniert ist, als die Blutgefäßposition (61) des Objekts.

10. Verfahren zum Schätzen von Bio-Informationen eines Benutzers, wobei das Verfahren umfasst:
Messen (1110) durch einen Impulswellensensor (110) einer Vielzahl von Impulswellensignalen von einem Objekt;
Beziehen (1120) durch einen Positionssensor (120) von Sensorpositionsinformationen, die eine Sensorposition an dem Objekt für jedes aus der Vielzahl von Impulswellensignalen auf der Basis des Impulswellensensors (110) identifizieren, der jedes aus der Vielzahl von Impulswellensignalen misst;
Schätzen (1130) durch einen Prozessor (130) von ersten Bio-Informationen an jeder Sensorposition auf der Basis von jedem aus der Vielzahl von Impulswellensignalen und
Schätzen (1140) durch den Prozessor von zweiten Bio-Informationen auf der Basis einer Blutgefäßposition des Objekts, jeder Sensorposition und der ersten Bio-Informationen an jeder Sensorposition, wobei
das Schätzen (1140) der zweiten Bio-Informationen das Generieren einer Kalibrierkurve (62) umfasst, indem erste geschätzte Bio-Informationswerte an jeder Sensorposition gegenüber einem relativen Abstand jeder Sensorposition von der Blutgefäßposition des Objekts geplottet werden und eine Kurvenanpassung durchgeführt wird, wobei der zweite geschätzte Bio-Informationswert auf der Basis der Kalibrierkurve (62) bezogen (1150) wird.

11. Verfahren nach Anspruch 10, das des Weiteren das Bestimmen (1240) von einer aus einer Vielzahl von virtuellen Blutgefäßpositionen als eine Blutgefäßposition des Objekts auf der Basis der ersten Bio-Informationen an jeder Sensorposition umfasst.

12. Verfahren nach Anspruch 10 oder 11, das angepasst ist, um die Vorrichtung (100) zum Schätzen von Bio-Informationen des Benutzers nach einem der Ansprüche 1 bis 9 zu betreiben.

## Revendications

1. Appareil (100) pour estimer les informations biologiques d'un utilisateur, l'appareil (100) comprenant :
un capteur d'ondes de pouls (110) configuré pour mesurer (1110) une pluralité de signaux d'ondes de pouls provenant d'un objet de l'utilisateur ;
un capteur de position (120) configuré pour obtenir (1120) des informations de position de capteur identifiant une position de capteur sur l'objet pour chacun de la pluralité de signaux d'ondes de pouls, sur la base du capteur d'ondes de pouls (110) mesurant chacun de la pluralité de signaux d'ondes de pouls ; et
un processeur (130) configuré pour :
estimer (1130) de premières informations biologiques à chaque position de capteur sur la base de chacun de la pluralité de signaux d'ondes de pouls ;
générer un graphique d'étalonnage (62) en traçant les premières valeurs d'informations biologiques estimées à chaque position de capteur en fonction d'une distance relative de chaque position de capteur par rapport à une position de vaisseau sanguin (61) de l'objet ;
sur la base d'un ajustement de courbe, obtenir (1140) une deuxième valeur d'informations biologiques estimée sur la base du graphique d'étalonnage (62) ; et
obtenir (1150) une valeur d'informations biologiques à un point, correspondant à la position de vaisseau sanguin (61) de l'objet dans le graphique d'étalonnage (62), en tant que deuxièmes informations biologiques.

2. Appareil (100) selon la revendication 1, dans lequel, sur la base de l'objet étant en contact avec le capteur d'ondes de pouls (110), le capteur de position (120) est en outre configuré pour obtenir (1120) les informations de position de capteur sur la base d'une image de l'objet qui est capturée par un dispositif de capture externe ou sur la base d'informations de position de mesure prédéfinies du capteur d'ondes de pouls (110).

3. Appareil (100) selon la revendication 1 ou 2, dans lequel le capteur de position (120) comprend un capteur d'empreintes digitales configuré pour obtenir une image d'empreintes digitales, et dans lequel le capteur de position (120) est en outre configuré pour obtenir (1120) les informations de position de capteur sur la base de l'image d'empreintes digitales obtenue par le capteur d'empreintes digitales sur la base de l'objet étant en contact avec le capteur d'ondes de pouls (110), ou
comprenant en outre un capteur de position des vaisseaux sanguins (310) configuré pour obtenir les informations de position des vaisseaux sanguins de l'objet sur la base d'au moins une image optique, une image ultrasonique, une image d'imagerie par résonance magnétique (IRM) et une image photoacoustique de l'objet obtenues par un dispositif externe, ou
comprenant en outre un capteur de position des vaisseaux sanguins (310) qui inclut en outre un capteur ultrasonique configuré pour transmettre une onde ultrasonique à l'objet et recevoir un signal réfléchi par l'objet, et obtenir les informations de position des vaisseaux sanguins de l'objet sur la base d'une image ultrasonique obtenue par le capteur ultrasonique.

4. Appareil (100) selon l'une des revendications 1 à 3, comprenant en outre :
un capteur de force (210) configuré pour mesurer une force appliquée par l'objet au capteur d'ondes de pouls (110) ; ou
un capteur de pression (210) configuré pour mesurer une pression appliquée par l'objet au capteur d'ondes de pouls (110).

5. Appareil (100) selon la revendication 4, dans lequel le processeur (130) est en outre configuré pour :
générer un oscillogramme basé sur chacun de la pluralité de signaux d'ondes de pouls et la force mesurée par le capteur de force (210) ou la pression mesurée par le capteur de pression (210) ; et
estimer les premières informations biologiques à chaque position de capteur en utilisant l'oscillogramme.

6. Appareil (100) selon l'une des revendications 1 à 5, dans lequel les informations biologiques comprennent un ou plusieurs éléments parmi la pression artérielle, l'âge vasculaire, la rigidité artérielle, la forme d'onde de pression aortique, la compliance vasculaire, l'indice de stress, le niveau de fatigue, l'âge de la peau et l'élasticité de la peau.

7. Appareil (100) selon l'une des revendications 1 à 6, dans lequel le processeur (130) est en outre configuré pour déterminer (1240) l'une d'une pluralité de positions virtuelles de vaisseaux sanguins en tant que position de vaisseau sanguin de l'objet sur la base des premières informations biologiques à chaque position de capteur.

8. Appareil (100) selon la revendication 7, dans lequel, sur la base d'une différence entre les premières valeurs d'informations biologiques à chaque position de capteur, le processeur (130) est en outre configuré pour déterminer (1240) l'une de la pluralité de positions virtuelles de vaisseaux sanguins en tant que position de vaisseau sanguin (61) de l'objet.

9. Appareil (100) selon la revendication 8, dans lequel une position virtuelle de vaisseau sanguin est définie pour chacun d'une pluralité de groupes qui sont pré-classifiés sur la base de la différence entre les premières valeurs d'informations biologiques à chaque position de capteur obtenues à partir d'une pluralité d'utilisateurs, ou
dans lequel le processeur (130) est en outre configuré pour :
déterminer un groupe, auquel la différence appartient, parmi la pluralité de groupes ; et
déterminer une position virtuelle de vaisseau sanguin, prédéfinie pour le groupe déterminé, en tant que position de vaisseau sanguin (61) de l'objet.

10. Procédé pour estimer des informations biologiques d'un utilisateur, le procédé comprenant :
la mesure (1110) par un capteur d'ondes de pouls (110), d'une pluralité de signaux d'ondes de pouls provenant d'un objet ;
l'obtention (1120) par un capteur de position (120), d'informations de position de capteur identifiant une position de capteur sur l'objet pour chacun de la pluralité de signaux d'ondes de pouls, sur la base du capteur d'ondes de pouls (110) mesurant chacun de la pluralité de signaux d'ondes de pouls ;
l'estimation (1130) par un processeur (130), de premières informations biologiques à chaque position de capteur sur la base de chacun de la pluralité de signaux d'ondes de pouls ; et
estimation (1140) par le processeur, des deuxièmes informations biologiques basées sur la position de vaisseau sanguin de l'objet à chaque position de capteur, et des premières informations biologiques à chaque position de capteur, dans lequel l'estimation (1140) des deuxièmes informations biologiques comprend la génération d'un graphique d'étalonnage (62) en traçant les premières valeurs d'informations biologiques estimées à chaque position de capteur en fonction d'une distance relative de chaque position de capteur par rapport à la position de vaisseau sanguin de l'objet, et en effectuant un ajustement de courbe, l'obtention (1150) d'une deuxième valeur d'informations biologiques estimée basée sur le graphique d'étalonnage (62).

11. Procédé selon la revendication 10 comprenant en outre la détermination (1240) d'une parmi une pluralité de positions virtuelles de vaisseaux sanguins en tant que position de vaisseau sanguin de l'objet, sur la base des premières informations biologiques à chaque position de capteur.

12. Procédé selon la revendication 10 ou 11 adapté pour faire fonctionner l'appareil (100) pour estimer des informations biologiques de l'utilisateur selon l'une des revendications 1 à 9.
